# FASCICULE DE BREVET EUROPEEN

(11) **EP 3 972 410 B1**
(45) Date de publication et mention de la délivrance du brevet: **07.08.2024**
(21) Numéro de dépôt: 20726164.5
(22) Date de dépôt: 20.05.2020
(51) Int. Cl.: A01N 1/02, A61K 35/19, C12N 5/078

(54) **COMPOSITION POUR LA CONSERVATION DES PLAQUETTES SANGUINES**
ZUSAMMENSETZUNG ZUR KONSERVIERUNG VON BLUTPLÄTTCHEN
COMPOSITION FOR THE PRESERVATION OF BLOOD PLATELETS

(30) Priorité: 20.05.2019 FR 1905268
(43) Date de publication de la demande: 30.03.2022
(73) Titulaire: Maco Pharma, 59420 Mouvaux (FR)
(72) Inventeur: HAVET, Coralie, 59200 Tourcoing (FR)
(74) Mandataire: Sayettat, Julien Christian
(86) Numéro de dépôt international: PCT/EP2020/064196
(87) Numéro de publication internationale: WO 2020/234417

(56) Documents cités:
- WO-A1-2016/164693
- WO-A1-2017/196798
- J.K. ARMSTRONG ET AL: "The Hydrodynamic Radii of Macromolecules and Their Effect on Red Blood Cell Aggregation", BIOPHYSICAL JOURNAL, vol. 87, no. 6, 1 December 2004 (2004-12-01), AMSTERDAM, NL, pages 4259 - 4270, XP055596614, ISSN: 0006-3495, DOI: 10.1529/biophysj.104.047746

## Description

L'invention concerne une composition pour la conservation des plaquettes, un procédé de préparation d'un produit plaquettaire à l'aide d'une telle composition et un produit plaquettaire comprenant des plaquettes et une telle composition.

Elle s'applique au domaine de la transfusion, et notamment à la préparation et la conservation des produits plaquettaires, tels que les concentrés plaquettaires.

Le sang total est constitué de deux types de composants : les cellules sanguines comprenant les globules rouges, les leucocytes et les plaquettes, et le plasma, liquide jaune pâle dans lequel les cellules sanguines sont en suspension.

Actuellement, on ne transfuse aux patients que les composants sanguins nécessaires à leur état. Par exemple, on ne transfuse que des concentrés plaquettaires aux patients atteints de thrombocytopénie, c'est-à-dire dont la teneur en plaquettes dans le sang est réduite.

Les concentrés plaquettaires sont obtenus par aphérèse ou à partir d'une ou plusieurs unités de sang total. Il existe en outre plusieurs méthodes de préparation des concentrés plaquettaires issus de sang total, parmi lesquelles on peut citer la méthode de préparation à partir de couches leucoplaquettaires (European Directorate for the Quality of Medicines. Healthcare (EDQM), Council of Europe: Guide to the préparation, use and quality assurance of blood components, (2017)).

Selon cette méthode de préparation, une unité de sang total est centrifugée de sorte à séparer le sang en trois couches : une couche de surnageant de plasma, une couche intermédiaire leucoplaquettaire comprenant un mélange de leucocytes, plaquettes, globules rouges et plasma, et une couche de culot de globules rouges. La couche leucoplaquettaire est séparée des autres couches. Entre quatre et six couches leucoplaquettaires ainsi séparées, sont ensuite réunies et re-suspendues dans du plasma ou dans une solution additive pour plaquettes. Ce mélange est à nouveau centrifugé (centrifugation douce) de sorte à obtenir une couche de surnageant de concentré plaquettaire et une couche de culot de globules rouges. A l'interface de ces deux couches, on trouve un anneau plaquettaire plus ou moins visible comportant un mélange concentré en plaquettes et des globules rouges. Le concentré plaquettaire est séparé, éventuellement déleucocyté par passage au travers d'un filtre à déleucocyter et conservé jusqu'à 4 à 7 jours à température ambiante. Lorsqu'on utilise une solution additive, le concentré plaquettaire final est constitué en général des plaquettes re-suspendues dans un mélange constitué de plasma (30-40%) et de solution additive (60-70%).

L'utilisation d'une solution additive pour plaquettes permet de réduire la quantité de plasma dans les concentrés plaquettaires et ainsi de réduire les risques de réactions transfusionnelles indésirables liées au plasma, tout en maintenant pendant la durée de conservation, une qualité de plaquettes comparable à celle des plaquettes préparées dans du plasma, sans solution additive.

Les solutions additives pour plaquettes comprennent généralement une solution saline avec un ou plusieurs composants choisis parmi l'acétate, le citrate, le phosphate, le gluconate, le carbonate, le potassium, le magnésium, le calcium et le glucose (Gulliksson, H. "Platelet storage media." Vox sanguinis 107.3 (2014): 205-212 ; Alhumaidan, Hiba, and Joseph Sweeney. "Current status of additive solutions for platelets." Journal of clinical apheresis 27.2 (2012): 93-98 ; Ringwald, Juergen, Robert Zimmermann, and Reinhold Eckstein. "The new génération of platelet additive solution for storage at 22 C: development and current expérience." Transfusion medicine reviews 20.2 (2006): 158-164).

Un exemple particulier d'une telle solution est la solution SSP+ commercialisée par Maco Pharma (Tourcoing, France). Cette solution comprend 69 mmol/L de chlorure de sodium, 5 mmol/L de chlorure de potassium, 1,5 mmo/L de chlorure de magnésium, 10 mmol/L de citrate de sodium, 26 mmol/L de phosphate (NaH2PO4 / Na2HPO4) et 30 mmol/L d'acétate de sodium.

Cependant, lorsque les concentrés plaquettaires sont préparés à l'aide d'une solution additive, on constate que le taux de récupération en plaquettes lors de la deuxième centrifugation est inférieur à celui des concentrés plaquettaires préparés avec du plasma seul (Zhang, Jerry G., et al. "Buffy-coat platelet variables and metabolism during storage in additive solutions or plasma." Transfusion 48.5 (2008): 847-856).

Pour tenter d'augmenter le rendement en plaquettes, le document EP 1 886 559 propose une solution additive pour plaquettes sanguines comprenant un agent augmentant la viscosité tel que l'hydroxyéthylamidon (HEA), le méthylcellulose, le polyéthylène glycol, le polyglycidol, la gélatine, les dextranes, l'albumine ou le dextrose. Ces agents augmentant la viscosité permettent à la solution additive d'atteindre une viscosité proche de celle du plasma, c'est-à-dire de 1,178 cp à 37°C. Ce document montre plus particulièrement qu'avec une solution additive comprenant de l'HEA ou du polyglycidol, il est possible de préparer des concentrés plaquettaires à partir de couches leucoplaquettaires avec un rendement plaquettaire sensiblement équivalent à celui obtenu pour des concentrés plaquettaires préparés avec du plasma. Les autres molécules citées, et notamment les polyéthylène glycols et les dextranes, n'ont pas été testées.

L'invention propose une autre solution pour augmenter le rendement en plaquettes lors de la préparation de concentrés plaquettaires dérivés de couches leucoplaquettaires avec une solution additive.

Par ailleurs, il est connu d'utiliser des agents de sédimentation des globules rouges pour séparer les globules blancs et autres cellules d'intérêt du sang et plus particulièrement du sang de cordon ombilical. Par exemple, dans le document US 5 789 147, le sang total anticoagulé est mis en contact avec de l'HEA, puis centrifugé de sorte à obtenir un surnageant enrichi en globules blancs et un culot de globules rouges.

Dans la demande WO 2017/196798A1, il est décrit une méthode sans centrifugation pour préparer un plasma riche en plaquettes par mise en contact du sang total anticoagulé avec un agent induisant la formation de Rouleaux, tel que l'HEA.

Il ressort de la littérature que ces agents de sédimentation des globules rouges sont généralement utilisés avec du sang total pour enrichir les fractions de leucocytes.

Enfin, le document WO 2016/164693 propose une solution pour conserver les plaquettes, celle-ci comprenant de 0,1 à 5% d'un polyéthylène glycol de bas poids moléculaire (entre 100 Da et 500 Da) afin d'éviter l'activation des plaquettes durant leur conservation.

Selon un premier aspect, l'invention propose une composition sans plasma pour la conservation des plaquettes comprenant une solution additive contenant (a) du chlorure de sodium et (b) un composé acétate ou un composé citrate ou une combinaison de ceux-ci, ladite composition comprenant en outre un agent d'agrégation des globules rouges choisi parmi le dextrane ayant un poids moléculaire de 50 kDa ou plus, le polyvinylpyrrolidone ayant un poids moléculaire de 40 kDa ou plus et le polyéthylène glycol ayant un poids moléculaire de 20 kDa ou plus ou une combinaison de ceux-ci.

Selon un deuxième aspect, l'invention porte sur un procédé de préparation d'un produit de plaquettes comprenant les étapes suivantes :
- fournir une composition selon le premier aspect,
- combiner ladite composition à une ou plusieurs couches leucoplaquettaires, de sorte à obtenir un mélange de couche(s) leucoplaquettaire(s) et de composition,
- centrifuger le mélange de couche(s) leucoplaquettaire(s) et de composition ainsi obtenu, et
- séparer le concentré plaquettaire du mélange ayant été centrifugé.

Selon un troisième aspect, l'invention divulgue un produit plaquettaire comprenant des plaquettes et une composition selon le premier aspect de l'invention.

D'autres objets et avantages apparaîtront au cours de la description qui suit.

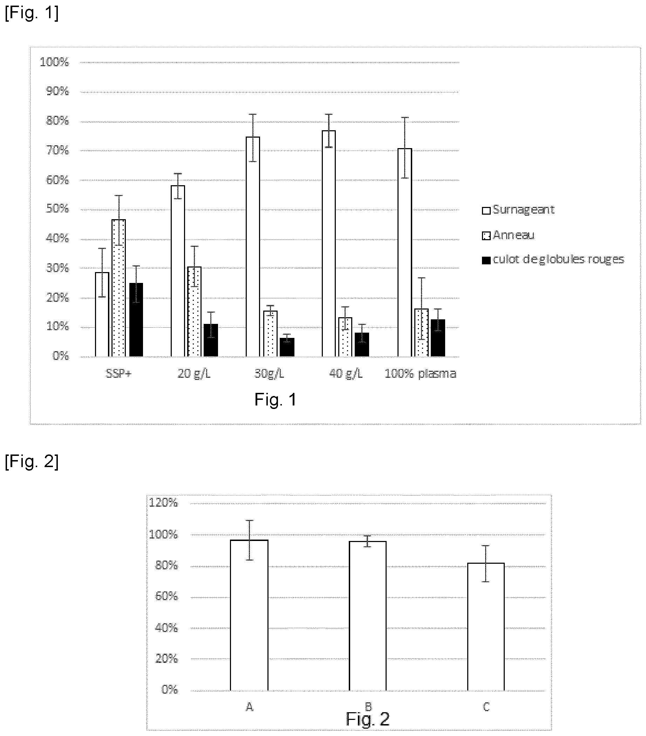

[Fig.1] représente la répartition en pourcentage et après centrifugation du nombre de plaquettes dans les différentes phases d'une couche leucoplaquettaire combinée à la solution additive SSP+, la solution additive SSP+ comprenant 20, 30 ou 40 g/L de dextran 70, ou sans solution additive (100% plasma).
[Fig. 2] représente le rendement en plaquettes d'une préparation d'un concentré plaquettaire issu d'un mélange de cinq couches leucoplaquettaires combinées avec la solution additive SSP+ comprenant 30 g/L de dextran 70 (A), du plasma (B) ou la solution additive SSP+ seule (C).

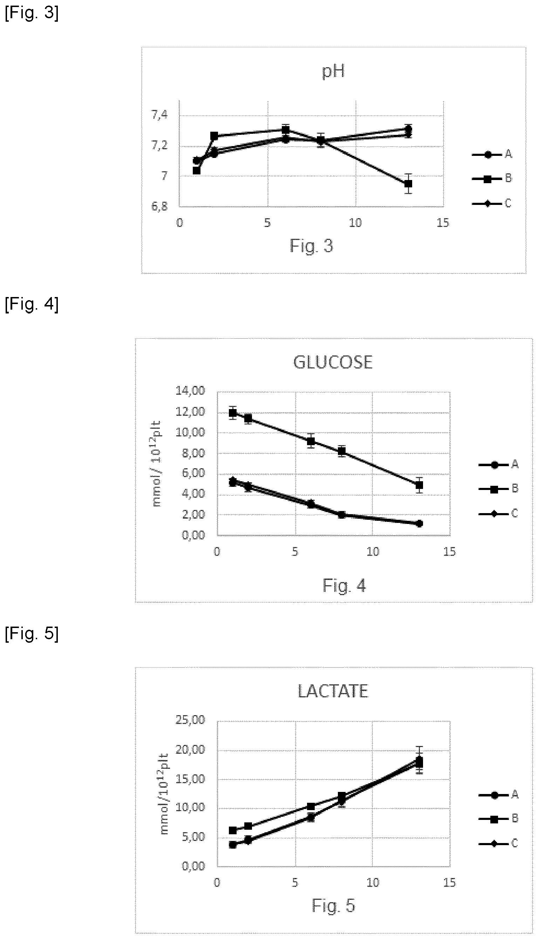

[Fig. 3] représente le pH après 1, 2, 6, 8 et 13 jours de conservation d'un concentré plaquettaire issu d'un mélange de cinq couches leucoplaquettaires combinées avec la solution additive SSP+ comprenant 30 g/L de dextran 70 (A), du plasma (B) ou la solution additive SSP+ seule (C).
[Fig. 4] représente la consommation en glucose après1, 2, 6, 8 et 13 jours de conservation d'un concentré plaquettaire issu d'un mélange de cinq couches leucoplaquettaires combinées avec la solution additive SSP+ comprenant 30 g/L de dextran 70 (A), du plasma (B) ou la solution additive SSP+ seule (C).
[Fig. 5] représente la production de lactate après 1, 2, 6, 8 et 13 jours de conservation, d'un concentré plaquettaire issu d'un mélange de cinq couches leucoplaquettaires combinées avec la solution additive SSP+ comprenant 30 g/L de dextran 70 (A), du plasma (B) ou la solution additive SSP+ seule (C).

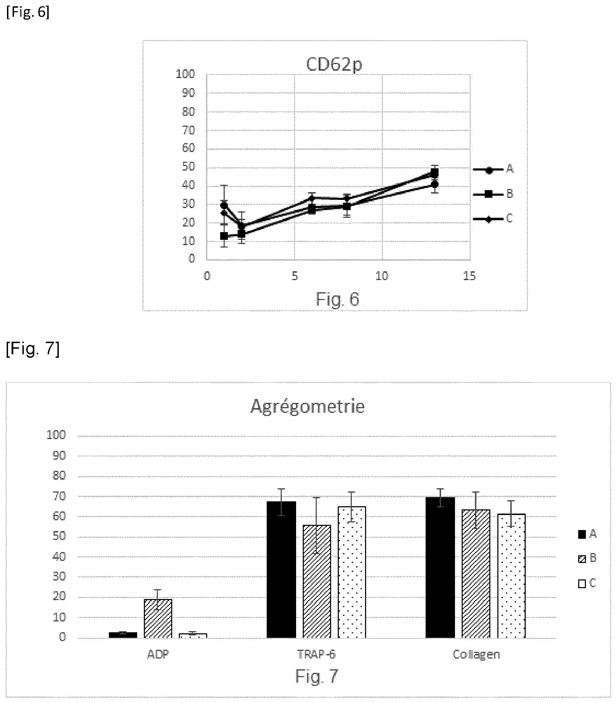

[Fig. 6] représente le pourcentage après 1, 2, 6, 8 et 13 jours de conservation, de cellules positives au marqueur CD62p membranaire, d'un concentré plaquettaire issu d'un mélange de cinq couches leucoplaquettaires combinées à la solution additive SSP+ comprenant 30 g/L de dextran 70 (A), du plasma (B) ou la solution additive SSP+ seule (C).
[Fig. 7] représente le pourcentage d'agrégation maximal à 5 jours en réponse à l'agoniste ADP, TRAP-6 ou collagène, concentré plaquettaire issu d'un mélange de cinq couches leucoplaquettaires combinées avec la solution additive SSP+ comprenant 30 g/L de dextran 70 (A), du plasma (B) ou la solution additive SSP+ seule (C).

L'invention concerne une composition pour la conservation des plaquettes, destinée à être combinée à un produit plaquettaire tel qu'un concentré de plaquettes. Cette composition favorise la récupération des plaquettes lors de la préparation d'un concentré plaquettaire à partir d'un mélange de couches leucoplaquettai res.

La composition est une composition synthétique qui comprend d'une part une solution additive et d'autre part un agent d'agrégation des globules rouges. Par « composition synthétique », on entend une composition n'ayant pas de correspondance dans la nature, produite de manière artificielle en combinant les différents composants la constituant.

La composition est exempte de protéines plasmatiques. La composition ne comprend pas de plasma.

La solution additive est une solution synthétique aqueuse. La solution additive contient du chlorure de sodium et l'un au moins des composants suivants : un composé citrate, un composé acétate, un composé phosphate, un composé bicarbonate, un composé gluconate, un composé magnésium, un composé potassium, un composé calcium, du glucose, ou une combinaison de ceux-ci.

Les composés de type sel d'acide sont notamment le citrate de sodium, l'acétate de sodium, une combinaison d'hydrogénophosphate de sodium et de dihydrogénophosphate de sodium, le bicarbonate de sodium et le gluconate de sodium.

Les composés magnésium, potassium et calcium sont notamment les composés ioniques de chlorure de magnésium, chlorure de potassium et chlorure de calcium.

En particulier, la solution additive contient (a) du chlorure de sodium et (b) un composé acétate ou un composé citrate ou une combinaison de ceux-ci.

Par exemple, la solution additive comprend (a) de 40 à 150 mM de chlorure de sodium et (b) de 10 à 50 mM d'un composé acétate tel que l'acétate de sodium et/ou de 5 à 20 mM d'un composé citrate tel que le citrate de sodium.

.Plus particulièrement, la solution additive comprend en outre un composé phosphate.

Par exemple la solution additive comprend de 0,05 à 50 mM de phosphate de sodium tel que le phosphate de sodium monobasique ou phosphate de sodium dibasique.

Encore plus particulièrement, la solution additive comprend en outre un composé magnésium et composé potassium.

Par exemple, la solution additive comprend de 0,1 à 5 mM de chlorure de magnésium et de 5 à 20 mM de chlorure de potassium.

Dans un autre exemple, la solution additive comprend de 1 à 40 mM de glucose et/ou de 0,01 mM à 4 mM de chlorure de calcium.

Notamment, la solution additive contient, outre le chlorure de sodium, de l'acétate de sodium et du citrate de sodium. Plus particulièrement, la solution additive comprend aussi de l'hydrogénophosphate de sodium et du dihydrogénophosphate de sodium. Et encore plus particulièrement, la solution additive comprend aussi chlorure de potassium et du chlorure de magnésium.

Par exemple, la solution additive est la solution SSP+ commercialisée par Maco Pharma.

Des exemples connus de solutions additives sont décrits dans les tableaux 1 et 2 ci-dessous.

**[Tableau 1]**

| Nom | PAS-A | PAS-B | PAS-C | PAS D |
|---|---|---|---|---|
| | PAS-I | PAS-II | PAS-III | |
| | PAS-1 | PAS-2 | | |
| Désignation commerciale | plasmalyte | T-Sol SSP | InterSol | Composol |
| Composition (mM) | | | | |
| Chlorure de sodium | 90 | 116 | 77 | 90 |
| Acétate de sodium | 27 | 30 | 30 | 27 |
| Citrate de sodium | - | 10 | 10 | 11 |
| Acide citrique | - | - | - | |
| Chlorure de potassium | 5 | - | - | 5 |
| Chlorure de magnésium | 3 | - | - | 1,5 |
| Chlorure de calcium | - | - | - | - |
| NaH₂PO₄/NaHPO₄ | - | - | 26 | - |
| Gluconate de sodium | 23 | - | - | 23 |
| Glucose | - | - | - | - |
| Carbonate de sodium | - | - | - | - |

**[Tableau 2]**

| Nom | PAS-E | PAS-Fa | PAS-G | BRS-A |
|---|---|---|---|---|
| | PAS-IIIM | | M-Sol | |
| | PAS-5 | | | |
| Désignation commerciale | SSP+ T-PAS+ | Isoplate | | |
| Composition (mM) | | | | |
| Chlorure de sodium | 69 | 141 | 110 | 95 |
| Acétate de sodium | 30 | 27 | 15 | - |
| Citrate de sodium | 10 | - | 10 | 4 |
| Acide citrique | | | | 2 |
| Chlorure de potassium | 5 | 5 | 5 | 4 |
| Chlorure de magnésium | 1,5 | 3 | 3 | 1 |
| Chlorure de calcium | - | - | - | 1.5 |
| NaH₂PO₄/NaHPO₄ | 26 | 1 | 4 | - |
| Gluconate de sodium | - | 23 | - | - |
| Glucose | - | - | 30 | 6 |
| Carbonate de sodium | - | - | 12 | 27 |

Ces solutions additives sont classiquement utilisées pour préparer et conserver des produits plaquettaires tels que des concentrés plaquettaires. Par exemple, pour préparer un concentré plaquettaire issu de sang total à partir de couches leucoplaquettaires, il est connu de centrifuger au moins une unité de sang total collectée d'un donneur, d'en séparer la couche leucoplaquettaire comprenant les plaquettes, de combiner entre quatre et sept couches plaquettaires avec une telle solution additive, de centrifuger à nouveau le mélange de couches leucoplaquettaires et de solution additive, et finalement de séparer le concentré plaquettaire comprenant les plaquettes en suspension dans un mélange de plasma et de solution additive.

Si ce mode de préparation est avantageux en ce qu'il réduit notamment la quantité de plasma dans le produit final, il est apparu que le rendement en plaquettes, c'est-à-dire le rapport entre le nombre de plaquettes présentes dans le concentré plaquettaire final et le nombre cumulé de plaquettes présentes dans les couches leucoplaquettaires de départ, est inférieur au rendement en plaquettes obtenu en utilisant du plasma à la place de la solution additive.

La composition pour la conservation des plaquettes de l'invention permet d'améliorer ce rendement en plaquettes par rapport à une solution additive classique.

Pour cela, la composition comprend, outre la solution additive telle que décrite ci-dessus, un agent d'agrégation des globules rouges choisi parmi le dextrane ayant un poids moléculaire de 50 kDa ou plus, le polyvinylpyrrolidone ayant un poids moléculaire de 40 kDa ou plus et le polyéthylène glycol ayant un poids moléculaire de 20 kDa ou plus ou une combinaison de ceux-ci.

L'enseignement de l'art antérieur, par exemple tel qu'illustré dans le document Zhang, J. G., et al. "Comparison of a novel viscous platelet additive solution and plasma: préparation and in vitro storage parameters of buffy-coat-derived platelet concentrates." Vox sanguinis 94.4 (2008): 299-305., propose, pour augmenter ce rendement plaquettaire, de modifier la solution additive afin d'atteindre une viscosité équivalente à celle du plasma à 37°C. Après plusieurs tests sur des solutions additives combinées avec différents composants afin d'atteindre une viscosité proche de celle du plasma à 37°C, la demanderesse s'est rendue compte que la viscosité de la solution additive n'était pas le facteur prédominant influençant le rendement plaquettaire (voir l'exemple 1 ci-dessous). Un facteur plus influençant serait la capacité de la composition à sédimenter les globules rouges.

Dans le plasma et en condition statiques, les globules rouges s'empilent les uns sur les autres pour former des « rouleaux » linéaires qui s'associent ensuite entre eux pour former des agrégats érythrocytaires plus volumineux. Ce phénomène est réversible : les agrégats se dispersent par action mécanique ou lorsque les forces de cisaillement augmentent.

L'agrégation des globules rouges est favorisée par le fibrinogène, protéine présente naturellement dans le plasma, mais aussi par des macromolécules exogènes telles que la gélatine ou certains polyéthylène glycols, polyvinylpyrroldidones et dextranes.

La demanderesse s'est rendue compte qu'en combinant la solution additive avec un agent d'agrégation des globules rouges tel que le dextrane ayant un poids moléculaire de 50 kDa ou plus, le polyvinylpyrrolidone ayant un poids moléculaire de 40 kDa ou plus et le polyéthylène glycol ayant un poids moléculaire de 20 kDa ou plus ou une combinaison de ceux-ci, le rendement plaquettaire est équivalent à celui obtenu en utilisant du plasma.

Ces macromolécules particulières amorcent la séparation des phases dans le mélange de couche(s) leucoplaquettaire(s) et de solution additive supplémentée avec ces macromolécules. Cette séparation est ensuite accentuée par la centrifugation finale.

Le dextrane est un polymère polysaccharidique obtenu par fermentation bactérienne ou par synthèse. Il est composé d'unités glucosidiques reliées par des liaisons alpha 1-6. Les dextranes dont le poids moléculaire va de 1000 Da à 2 000 000 Da, sont généralement identifiés selon leur poids moléculaire. Par exemple, le dextran 40 correspond à un dextrane ayant un poids moléculaire moyen de 40 000 Da.

Le polyvinylpyrrolidone (PVP) est un polymère synthétique obtenu par polymérisation de la N-vinylpyrrolidone. Les polyvinylpyrrolidones sont disponibles dans le commerce avec un poids moléculaire moyen allant de 2 000 Da à 3 000 000 Da.

Le polyéthylène glycol (PEG) est un polymère synthétique constitué d'unité d'oxyde d'éthylène. Les polyéthylène glycols ont un poids moléculaire généralement inférieur à 100 000 Da et sont également identifiés par leur poids moléculaire. Par exemple, le PEG 20 000 ou PEG 20M possède un poids moléculaire moyen de 20 000 Da.

Ces macromolécules ont le point commun de favoriser ou d'inhiber l'agrégation des globules rouges dans le plasma selon leur poids moléculaire. En effet, Armstrong et al. ont montré que les macromolécules telles que le dextrane ayant un poids moléculaire supérieur à environ 40 000, le polyvinylpyrrolidone ayant un poids moléculaire supérieur à environ 20 000 et le polyoxyéthylène ayant un poids moléculaire supérieur à environ 15 000, qui possèdent un rayon hydrodynamique (Rh) supérieur à 4, favorisent l'agrégation des globules rouges en suspension dans du plasma. (Armstrong, Jonathan K., et al. "The hydrodynamic radii of macromolecules and their effect on red blood cell aggregation." Biophysical journal 87.6 (2004): 4259-4270).

En particulier, l'agent d'agrégation présent dans la composition est le polyéthylène glycol ayant un poids moléculaire de 20 kDa (PEG 20M). Plus particulièrement, la concentration en PEG 20M dans la composition pour la conservation des plaquettes va de 20 à 40 g/L.

En variante, l'agent d'agrégation des globules rouges est le dextrane ayant un poids moléculaire de 70 kDa (dextran 70).

Plus particulièrement, la concentration en dextran 70 dans la composition pour la conservation des plaquettes va de 20 à 80 g/L, notamment de 30 à 50 g/L.

Une concentration inférieure à 20 g/L ou supérieure à 80 g/L en dextran 70 dans la composition n'a pas d'effet favorisant l'agrégation des globules rouges dans une couche leucoplaquettaire.

La composition de l'invention pour conserver les plaquettes est exempte de plaquettes. Elle est destinée à être combinée à un produit plaquettaire, tel qu'un concentré plaquettaire, lui-même destiné à être injecté à un patient. A cet effet, la composition possède un pH compris entre 6,5 et 8, particulièrement 7 et 7,8.

En outre, la composition de l'invention possède une osmolarité proche de celle du sérum sanguin, notamment comprise en 270 et 320 mOsm/kg.

La composition pour la conservation des plaquettes comprend avantageusement la solution additive combinée de fabrication à l'agent d'agrégation des globules rouges.

Ainsi, la composition pour la conservation des plaquettes est notamment conditionnée dans un récipient unique tel qu'une poche souple.

En variante, la composition se présente sous la forme d'un kit comprenant d'une part la solution additive conditionnée dans un premier récipient tel qu'une poche souple, et d'autre part l'agent d'agrégation des globules rouges conditionné dans un second récipient.

En vue d'une application à usage thérapeutique, la composition est stérile. Notamment, la composition est stérilisée par vapeur ou irradiation par rayonnement gamma.

Selon le deuxième aspect, l'invention concerne un procédé de préparation d'un produit de plaquettes à l'aide de la composition décrite ci-dessus comprenant un agent d'agrégation des globules rouges.

Le produit de plaquettes est notamment un concentré plaquettaire comprenant plus de 2.10¹¹ plaquettes.

Le procédé de préparation comprend les étapes suivantes :
- fournir une composition selon le premier aspect de l'invention,
- combiner ladite composition à une ou plusieurs couches leucoplaquettaires, de sorte à obtenir un mélange de couches leucoplaquettaires et de composition,
- centrifuger le mélange de couches leucoplaquettaires et de composition ainsi obtenu, et
- séparer le concentré plaquettaire du mélange ayant été centrifugé.

Lorsque la composition pour la conservation des plaquettes se présente sous la forme d'un kit en deux parties, à savoir d'une part la solution additive et d'autre part l'agent d'agrégation des globules rouges, l'étape consistant à combiner la composition avec une ou plusieurs couche(s) leucoplaquettaire(s) consiste à combiner d'abord la solution additive et l'agent d'agrégation, puis à combiner la composition ainsi reconstituée avec la ou les couche(s) leucoplaquettaire(s).

En variante, la solution additive est d'abord combinée à la ou les couche(s) leucoplaquettaire(s), puis le mélange est ensuite combiné avec l'agent d'agrégation des globules rouges.

En autre variante, l'agent d'agrégation des globules rouges est d'abord combiné avec la ou les couche(s) leucoplaquettaire(s), et ce mélange est ensuite combiné à la solution additive.

Chacune des couches leucoplaquettaires est obtenue par centrifugation d'une unité de sang total préalablement collectée d'un donneur, afin d'obtenir une couche de plasma, une couche leucoplaquettaire et une couche de globules rouges. La couche leucoplaquettaire est séparée des autres couches, par exemple à l'aide d'une presse séparation.

Après combinaison de la composition avec la ou les couche(s) leucoplaquettaire(s), le mélange est à nouveau centrifugé pour obtenir une couche surnageante de concentré plaquettaire et un culot de globules rouges.

L'étape de séparation du concentré plaquettaire est réalisée notamment à l'aide d'une presse de séparation.

Avantageusement, le procédé comprend en outre l'étape de filtrer le concentré plaquettaire pour éliminer les leucocytes.

La filtration est notamment réalisée lors de la séparation du concentré plaquettaire. Un exemple de filtre à déleucocyter les concentrés plaquettaires est décrit dans le document FR 300 3764 A1.

Selon une réalisation, le procédé de préparation comprend l'étape de conservation du concentré plaquettaire après sa séparation.

Le concentré plaquettaire est notamment conservé jusqu'à 4 à 7 jours à une température comprise entre 20°C et 24°C.

Selon un troisième aspect, l'invention porte sur un produit plaquettaire comprenant des plaquettes et une composition selon le premier aspect de l'invention.

En particulier, le produit plaquettaire est un concentré plaquettaire obtenu par le procédé selon le deuxième aspect de l'invention.

En particulier, le produit plaquettaire comprend du plasma dans un volume allant de 20% à 50% du volume de produit plaquettaire, notamment entre 25% et 40% du volume de produit plaquettaire.

Dans le produit plaquettaire, le volume de composition pour la conservation des plaquettes varie de 50% à 80% du volume de produit plaquettaire, notamment de 60% à 75% du volume de produit plaquettaire.

Notamment, le produit est à usage thérapeutique et contient une quantité de plaquettes supérieure à de 2.10¹¹.

Exemple 1 : Impact de différentes molécules sur la sédimentation des globules rouges dans une couche leucoplaquettaire.

Cet essai est basé sur la mesure de l'ESR (Erythrocyte Sédimentation Rate) à l'aide du dispositif de Westergren.

L'étude a été réalisée sur un échantillon de couche leucoplaquettaire dans des conditions de rapport solution additive/couche leucoplaquettaire comparable à celui utilisé pour la préparation classique d'un concentré plaquettaire, à savoir cinq couches leucoplaquettaires pour 300 mL de solution additive. Dans un tube de 5 mL, on transfère 1,6 mL d'une couche leucoplaquettaire préparée le jour même, puis 2,4 mL de solution test ou de plasma.

Les solutions tests, détaillées dans le tableau 3, sont constituées de la solution additive SSP+ supplémentée avec différentes molécules dans une concentration suffisante pour obtenir une solution ayant une viscosité proche de celle du plasma, à savoir 1,178 mPa.s.

**[Tableau 3]**

| Solution | Concentration dans la SSP+ (g/L) | densité | Viscosité dynamique (mPa.s) |
|---|---|---|---|
| SSP+ | - | 1,021 | 0,749 |
| SSP+ / Dextran 40 | 28,656 | 1,037 | 1,180 |
| SSP+ / PEG 400 | 126,885 | 1,042 | 1,210 |
| SSP+ / Dextran 70 | 20,930 | 1,033 | 1,148 |
| Plasma | - | - | - |

Les résultats de sédimentation sont montrés dans le tableau 4 :

**[Tableau 4]**

| | Hauteur (mm) | | | |
|---|---|---|---|---|
| Solution | 15 min | 30 min | 60 min | 137 min |
| SSP+ | 0 | 0 | 1 | 2 |
| SSP+ / Dextran 40 | 0 | 1 | 2 | 5 |
| SSP+ / PEG 400 | 0 | 0 | 1 | 2 |
| SSP+ / Dextran 70 | 4 | 15 | 40 | 85 |
| Plasma | 3 | 10 | 34 | 69 |

On note que, seule le plasma et la solution contenant du dextran 70 présente une sédimentation significative comparée aux autres solutions testées. Les solutions contenant du dextran 40 ou du PEG ont une sédimentation équivalente à celle de la solution additive SSP+.

Bien que les solutions testées aient une viscosité équivalente et proche de celle du plasma, seule le dextran 70 montre une sédimentation similaire à celle du plasma.

Ce résultat tend à montrer que la viscosité n'est pas le facteur principal influençant la sédimentation des globules rouges dans la solution additive.

Exemple 2 : Impact de la concentration en PEG 20M dans la solution SSP+ sur la vitesse de sédimentation des globules rouges dans une couche leucoplaquettaire.

Le test de l'exemple 1 a été reproduit avec une solution SSP+ complémentée avec différentes concentrations de PEG 20M (0 à 30 g/L), comme indiqué dans le tableau 5.

**[Tableau 5]**

| | | Hauteur (mm) | | | |
|---|---|---|---|---|---|
| Solutions | Concentration (g/L) | 15 min | 30 min | 60 min | 120 min |
| SSP+ | - | 1 | 0 | 0 | 0,5 |
| SSP+/PEG 20M | 7,5 | 2 | 0 | 1 | 2 |
| SSP+/PEG 20M | 15 | 3 | 3 | 12 | 33 |
| SSP+/PEG 20M | 20 | 4 | 32 | 100 | 150 |
| SSP+/PEG 20M | 25 | 5 | 45 | 124 | 142 |
| SSP+/PEG 20M | 30 | 6 | 66 | 127 | 145 |

On note, qu'à partir d'une concentration de 20 g/L dans la solution, le PEG 20M a un effet significatif sur la sédimentation des globules rouges dans une couche leucoplaquettaire.

Exemple 3 : Impact de la concentration en dextran 70 dans la solution SSP+ sur la vitesse de sédimentation des globules rouges dans une couche leucoplaquettaire.

Le test décrit dans l'exemple 1 a été reproduit avec des solutions comprenant différentes concentrations de dextran 70, comme indiqué dans le tableau 6.

**[Tableau 6]**

| | | | Hauteur (mm) | | | |
|---|---|---|---|---|---|---|
| Solutions | Concentration (g/L) | Viscosité dynamique (mPa.s) | 15 min | 30 min | 60 min | 120 min |
| SSP+/dextran 70 | 5 | 0,838 | 0 | 1 | 1 | 3 |
| SSP+/dextran 70 | 10 | 0,932 | 0.5 | 2 | 3 | 5 |
| SSP+/dextran 70 | 20 | 1,152 | 4 | 11 | 30 | 74 |
| SSP+/dextran 70 | 25 | 1,262 | 6 | 18 | 44 | 90 |
| SSP+/dextran 70 | 30 | 1,407 | 6 | 24 | 57 | 112 |
| SSP+/dextran 70 | 40 | 1,705 | 12 | 40 | 88 | 143 |
| SSP+/dextran 70 | 50 | 2,022 | 13 | 38 | 96 | 144 |
| SSP+/dextran 70 | 60 | 2,429 | 10 | 30 | 80 | 140 |
| SSP+/dextran 70 | 80 | - | 7 | 12 | - | 40 |
| plasma | - | | 2 | 7 | 21 | 53 |

Plus la concentration en dextran 70 augmente, plus la sédimentation des globules rouges dans la couche leucoplaquettaire est important. Un palier semble atteint à partir d'une concentration de 40 g/L.

Le même test a été réalisé avec du dextran 40 dans une solution SSP+, avec une concentration variant de 5 à 80 g/L et aucun phénomène de sédimentation n'a été observé.

### Exemple 4 : Effet de la solution sur la récupération en plaquettes lors de la séparation des mélanges de couches leuco-plaquettaires

On a étudié la répartition plaquettaire dans les différentes couches après centrifugation d'une couche leuco-plaquettaire supplémentée en plasma, en solution SSP+ ou en solution SSP+ et dextran 70 (20 g/L ; 30 g/L ou 40 g/L).

Afin de limiter les variations en concentration plaquettaire entre donneurs, on a préparé un mélange de huit couches leucoplaquettaires ABO compatibles que l'on a redistribué dans huit poches.

On a ajouté à chacune de ces poches le plasma ou les différentes solutions SSP+ puis on a centrifugé les poches à 520g. Après centrifugation, on a séparé le surnageant, l'anneau plaquettaire et le culot. Pour chacune des couches, on a en déterminé le volume et la concentration en plaquettes afin de calculer le nombre de plaquettes.

Les résultats sont montrés sur la Fig. 1. On note que le pourcentage de plaquettes dans le surnageant avec la solution contenant 20 g/L de dextran 70 est significativement inférieur comparé aux solutions comprenant 30 et 40 g/L de dextran 70. Le pourcentage de plaquettes dans l'anneau est significativement supérieur avec une solution de 20 g/L de dextran 70 comparé avec la solution à 40 g/L. Le pourcentage de plaquettes dans le culot de globules rouges ne présente pas de différence significative selon les solutions testées.

En comparant avec le plasma et la solution SSP+, on observe que l'ajout de dextran 70 dans la solution SSP+ permet d'augmenter le pourcentage de plaquettes dans le surnageant. Les solutions comprenant 30 et 40 g/L de dextran 70 permettent d'obtenir une répartition plaquettaire semblable à celle du plasma.

### Exemple 5 : Test de conservation des plaquettes

On a préparé un concentré plaquettaire à partir d'un mélange de cinq couches leucoplaquettaire complémentées avec :
Solution A : 30% plasma et 70% solution SSP+/dextran 70 (30 g/L)
Solution B : 100% plasma
Solution C : 30% plasma et 70% solution SSP+.

Le concentré plaquettaire a été déleucocyté par passage au travers d'un filtre TXP (Maco Pharma, Tourcoing, France).

Les résultats, montrés sur la Fig. 2, montrent que l'utilisation de dextran 70 entraîne l'augmentation du rendement en plaquettes par rapport à l'utilisation de la solution SSP+ jusqu'à obtenir un rendement équivalent à celui obtenu avec le plasma.

Afin de contrôler la qualité des plaquettes au cours de leur durée de conservation, les paramètres suivants ont été mesurés entre J1 et J13 : pH, consommation de glucose, production de lactate, activation plaquettaire (%CD62p), et agrégométrie en réponse à trois agonistes (ADP, TRAP6 et collagène). Les résultats sont montrés sous forme de graphe sur les figures 4 à 7.

On ne note pas de différence significative entre des concentrés plaquettaires préparés avec la solution SSP+/dextran 70 ou la solution SSP+ seule sur le métabolisme et l'activation des plaquettes.

## Revendications

1. Composition sans plasma pour la conservation des plaquettes comprenant une solution additive contenant (a) du chlorure de sodium et (b) un composé acétate ou un composé citrate ou une combinaison de ceux-ci, ladite composition étant **caractérisée en ce qu'**elle comprend en outre un agent d'agrégation des globules rouges choisi parmi le dextrane ayant un poids moléculaire de 50 kDa ou plus, le polyvinylpyrrolidone ayant un poids moléculaire de 40 kDa ou plus et le polyéthylène glycol ayant un poids moléculaire de 20 kDa ou plus ou une combinaison de ceux-ci.

2. Composition selon la revendication 1, **caractérisée en ce que** la solution additive comprend en outre l'un des composants suivants ou une combinaison de ceux-ci : un composé phosphate, un composé bicarbonate, un composé gluconate, un composé magnésium, un composé potassium, un composé calcium, du glucose.

3. Composition selon l'une des revendications 1 ou 2, **caractérisée en ce que** la solution additive contient de l'acétate de sodium et du citrate de sodium.

4. Composition selon la revendication 3, **caractérisée en ce que** la solution additive comprend en outre de l'hydrogénophosphate de sodium et du dihydrogénophosphate de sodium.

5. Composition selon la revendication 4, **caractérisée en ce que** la solution additive comprend en outre du chlorure de potassium et du chlorure de magnésium.

6. Composition selon l'une quelconque des revendications 1 à 5, **caractérisée en ce que** l'agent d'agrégation est le polyéthylène glycol ayant un poids moléculaire de 20 kDa.

7. Composition selon l'une quelconque des revendications 1 à 5, **caractérisée en ce que** l'agent d'agrégation est le dextrane ayant un poids moléculaire de 70 kDa.

8. Composition selon la revendication 7, **caractérisée en ce que** la concentration de dextrane de poids moléculaire de 70 kDa dans la composition va de 20 à 80 g/L, notamment de 30 à 50 g/L.

9. Composition selon l'une quelconque des revendications 1 à 8, **caractérisée en ce qu'**elle possède un pH compris entre 6,5 et 8, particulièrement 7 et 7,8.

10. Composition selon l'une quelconque des revendications 1 à 9, **caractérisée en ce qu'**elle possède une osmolarité comprise en 270et 320 mOsm/kg.

11. Procédé de préparation d'un produit de plaquettes **caractérisé en ce qu'**il comprend les étapes suivantes :
- fournir une composition selon l'une quelconque des revendications 1 à 10,
- combiner ladite composition à une ou plusieurs couches leucoplaquettaires, de sorte à obtenir un mélange de couche(s) leucoplaquettaire(s) et de composition
- centrifuger le mélange de couche(s) leucoplaquettaire(s) et de composition ainsi obtenu,
- séparer le concentré plaquettaire du mélange ayant été centrifugé.

12. Procédé selon la revendication 11, **caractérisé en ce qu'**il comprend en outre l'étape de filtrer le concentré plaquettaire pour éliminer les leucocytes.

13. Procédé selon l'une des revendications 11 ou 12, **caractérisé en ce qu'**il comprend l'étape de conservation du concentré plaquettaire après sa séparation.

14. Produit plaquettaire comprenant des plaquettes et une composition selon l'une quelconque des revendications 1 à 10.

15. Produit plaquettaire selon la revendication 14 **caractérisé en ce qu'**il consiste en un concentré de plaquettes obtenu par le procédé selon l'une des revendications 11 à 13.

16. Produit plaquettaire selon l'une des revendications 14 ou 15, **caractérisé en ce qu'**il comprend du plasma dans un volume allant de 20% à 50% du volume de produit plaquettaire, notamment entre 25% et 40% du volume de produit plaquettaire.

## Patentansprüche

1. Plasmafreie Zusammensetzung zur Konservierung von Thrombozyten, eine Additivlösung umfassend, die (a) Natriumchlorid und (b) eine Acetatverbindung oder eine Citratverbindung oder eine Kombination davon enthält, wobei die Zusammensetzung **dadurch gekennzeichnet ist, dass** sie weiter ein Erythrozytenaggregationsmittel umfasst, ausgewählt aus Dextran mit einem Molekulargewicht von 50 kDa oder mehr, Polyvinylpyrrolidon mit einem Molekulargewicht von 40 kDa oder mehr, und Polyethylenglycol mit einem Molekulargewicht von 20 kDa oder mehr oder einer Kombination davon.

2. Zusammensetzung nach Anspruch 1, **dadurch gekennzeichnet, dass** die Additivlösung weiter eine der folgenden Komponenten oder eine Kombination davon umfasst: eine Phosphatverbindung, eine Bicarbonatverbindung, eine Gluconatverbindung, eine Magnesiumverbindung, eine Kaliumverbindung, eine Calciumverbindung, Glucose.

3. Zusammensetzung nach einem der Ansprüche 1 oder 2, **dadurch gekennzeichnet, dass** die Additivlösung Natriumacetat und Natriumcitrat enthält.

4. Zusammensetzung nach Anspruch 3, **dadurch gekennzeichnet, dass** die Additivlösung weiter Natriumhydrogenphosphat und Natriumdihydrogenphosphat umfasst.

5. Zusammensetzung nach Anspruch 4, **dadurch gekennzeichnet, dass** die Additivlösung weiter Kaliumchlorid und Magnesiumchlorid umfasst.

6. Zusammensetzung nach einem der Ansprüche 1 bis 5, **dadurch gekennzeichnet, dass** das Aggregationsmittel Polyethylenglykol mit einem Molekulargewicht von 20 kDa ist.

7. Zusammensetzung nach einem der Ansprüche 1 bis 5, **dadurch gekennzeichnet, dass** das Aggregationsmittel Dextran mit einem Molekulargewicht von 70 kDa ist.

8. Zusammensetzung nach Anspruch 7, **dadurch gekennzeichnet, dass** die Konzentration von Dextran mit einem Molekulargewicht von 70 kDa in der Zusammensetzung 20 bis 80 g/l, insbesondere 30 bis 50 g/l beträgt.

9. Zusammensetzung nach einem der Ansprüche 1 bis 8, **dadurch gekennzeichnet, dass** sie einen pH-Wert im Bereich zwischen 6,5 und 8, insbesondere 7 und 7,8, besitzt.

10. Zusammensetzung nach einem der Ansprüche 1 bis 9, **dadurch gekennzeichnet, dass** sie eine Osmolarität im Bereich von 270 und 320 mOsm/kg besitzt.

11. Verfahren zur Herstellung eines Thrombozytenprodukts, **dadurch gekennzeichnet, dass** es die folgenden Schritte umfasst:
- Bereitstellen einer Zusammensetzung nach einem der Ansprüche 1 bis 10,
- Kombinieren der Zusammensetzung mit einem oder mehreren Buffy-Coats, um eine Mischung aus Buffy-Coat(s) und Zusammensetzung zu erhalten,
- Zentrifugieren der so erhaltenen Mischung aus Buffy-Coat(s) und Zusammensetzung,
- Abtrennen des Thrombozytenkonzentrats von der zentrifugierten Mischung.

12. Verfahren nach Anspruch 11, **dadurch gekennzeichnet, dass** es weiter den Schritt des Filterns des Thrombozytenkonzentrats umfasst, um Leukozyten zu entfernen.

13. Verfahren nach einem der Ansprüche 11 oder 12, **dadurch gekennzeichnet, dass** es den Schritt des Konservierens des Thrombozytenkonzentrats nach seiner Abtrennung umfasst.

14. Thrombozytenprodukt, das Thrombozyten und eine Zusammensetzung nach einem der Ansprüche 1 bis 10 umfasst.

15. Thrombozytenprodukt nach Anspruch 14, **dadurch gekennzeichnet, dass** es aus einem Thrombozytenkonzentrat besteht, das über das Verfahren nach einem der Ansprüche 11 bis 13 erhalten wird.

16. Thrombozytenprodukt nach einem der Ansprüche 14 oder 15, **dadurch gekennzeichnet, dass** es Plasma in einem Volumen von 20 % bis 50 % des Volumens des Thrombozytenprodukts, insbesondere zwischen 25 % und 40 % des Volumens des Thrombozytenprodukts, umfasst.

## Claims

1. A plasma-free composition for platelet preservation comprising an additive solution containing (a) sodium chloride and (b) an acetate compound or a citrate compound or a combination thereof, said composition being **characterized in that** it further comprises a red blood cell aggregating agent selected from dextran having a molecular weight of 50 kDa or more, polyvinylpyrrolidone having a molecular weight of 40 kDa or more and polyethylene glycol having a molecular weight of 20 kDa or more or a combination thereof.

2. Composition according to claim 1, **characterized in that** the additive solution additionally comprises one of the following components or a combination thereof: a phosphate compound, a bicarbonate compound, a gluconate compound, a magnesium compound, a potassium compound, a calcium compound, glucose.

3. Composition according to one of claims 1 or 2, **characterized in that** the additive solution contains sodium acetate and sodium citrate.

4. Composition according to claim 3, **characterized in that** the additive solution additionally comprises sodium hydrogen phosphate and sodium dihydrogen phosphate.

5. Composition according to claim 4, **characterized in that** the additive solution additionally comprises potassium chloride and magnesium chloride.

6. Composition according to any one of claims 1 to 5, **characterized in that** the aggregating agent is polyethylene glycol having a molecular weight of 20 kDa.

7. Composition according to any one of claims 1 to 5, **characterized in that** the aggregating agent is dextran having a molecular weight of 70 kDa.

8. Composition according to claim 7, **characterized in that** the concentration of dextran with a molecular weight of 70 kDa in the composition ranges from 20 to 80 g/L, in particular from 30 to 50 g/L.

9. Composition according to any one of claims 1 to 8, **characterized in that** it has a pH of between 6.5 and 8, particularly 7 and 7.8.

10. Composition according to any one of claims 1 to 9, **characterized in that** it has an osmolarity of between 270 and 320 mOsm/kg.

11. Process for preparing a platelet product, **characterized in that** it comprises the following steps:
- providing a composition according to any one of claims 1 to 10,
- combining said composition with one or more leukoplaketin layers, so as to obtain a mixture of leukoplaketin layer(s) and composition
- centrifuging the mixture of leukoplaketin
layer(s) and composition thus obtained,
- separating the platelet concentrate from the centrifuged mixture.

12. Process according to claim 11, **characterized in that** it further comprises the step of filtering the platelet concentrate to remove leukocytes.

13. Process according to one of claims 11 or 12, **characterized in that** it comprises the step of preserving the platelet concentrate after its separation.

14. Platelet product comprising platelets and a composition according to any one of claims 1 to 10.

15. Platelet product according to claim 14, **characterized in that** it consists of a platelet concentrate obtained by the process according to one of claims 11 to 13.

16. Platelet product according to one of claims 14 or 15, **characterized in that** it comprises plasma in a volume ranging from 20% to 50% of the platelet product volume, in particular between 25% and 40% of the platelet product volume.
